(19) Europäisches Patentamt
European Patent Office
Office européen des brevets

(11) Numéro de publication: **0 468 946 B1**

# (12) FASCICULE DE BREVET EUROPEEN

(45) Date de publication de fascicule du brevet: **12.07.95**

(51) Int. Cl.[6]: **C12Q 1/37**, C07K 5/06, //(C12Q1/37,C12R1:03)

(21) Numéro de dépôt: **91870070.9**

(22) Date de dépôt: **26.04.91**

## (54) Procédé enzymatique pour la détermination d'antibiotiques à noyau beta-lactame.

(30) Priorité: **30.04.90 GB 9009692**

(43) Date de publication de la demande:
**29.01.92 Bulletin 92/05**

(45) Mention de la délivrance du brevet:
**12.07.95 Bulletin 95/28**

(84) Etats contractants désignés:
**AT BE CH DE DK ES FR GB GR IT LI LU NL SE**

(56) Documents cités:
**EP-A- 0 085 667**
**EP-A- 0 186 944**
**US-A- 2 824 863**
**US-A- 4 166 825**

**CHEMICAL ABSTRACTS, vol. 63, no. 3, 2 août 1965, Columbus, OH (US); G. LOWE et al., no. 3223e/**

**ANTIMICROBIAL AGENTS AND CHEMOTHERAPY, vol. 18, no. 4, octobre 1980; J.-M. FRERE et al., pp. 506-510/**

(73) Titulaire: **UCB-BIOPRODUCTS, S.A.**
**326, Avenue Louise**
**B-1050 Bruxelles (BE)**

(72) Inventeur: **Degelaen, Jacques**
**14 Avenue des taillis**
**B-1470 Genappe (BE)**
Inventeur: **Frere, Jean-Marie**
**9, Chemin du Sotrez**
**B-4550 nandrin (BE)**

(74) Mandataire: **Dusseldorp, Raymond**
**UCB S.A.**
**Département D.T.B.**
**33, rue d'Anderlecht**
**B-1620 Drogenbos (BE)**

EP 0 468 946 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention se rapporte à un nouveau procédé enzymatique, rapide et sensible pour la détermination d'antibiotiques à noyau β-lactame dans un liquide biologique. Elle concerne également une trousse d'essai utilisable pour la mise en oeuvre de ce procédé.

A l'heure actuelle, les antibiotiques sont très largement utilisés non seulement comme agents thérapeutiques dans le traitement des maladies infectieuses provoquées par les bactéries, mais également comme agents de conservation des aliments et comme additifs dans la nourriture des animaux pour stimuler la croissance. La nécessité se fait donc de plus en plus sentir de pouvoir détecter la présence d'antibiotiques, même en de très faibles concentrations, dans des liquides biologiques complexes, comme le lait, l'urine, le sang, le sérum, la salive, les extraits de viande, les liquides de fermentation, ou dans des milieux acqueux tamponnés.

Le cas de la production de lait en est un exemple. Il est bien connu en effet d'utiliser les pénicillines pour traiter certaines maladies infectieuses du bétail producteur de lait, comme par exemple la mastite. Or, pour des raisons médicales évidentes, le lait destiné à la consommation humaine doit, en principe, être exempt de toute trace d'antibiotiques. Par ailleurs, des concentrations en pénicilline de 0,005 U.I./ml ou moins peuvent avoir des incidences néfastes lors de la fabrication de produits dérivés du lait, comme le fromage, le yaourt, etc. En outre, dans certains pays, les normes légales imposent une concentration en antibiotique n'excédant pas 0,004 U.I./ml.

Il s'avère donc qu'il est nécessaire de pouvoir déterminer, rapidement et de manière précise, la concentration des pénicillines dans le lait produit par le bétail et ce, de préférence, directement sur place à la ferme.

Il existe depuis longtemps des procédés microbiologiques qui permettent de déterminer des concentrations relativement faibles d'antibiotiques β-lactames dans des liquides biologiques. Ces procédés sont basés sur la mesure de l'inhibition de la croissance de microorganismes sensibles aux antibiotiques, en présence d'un échantillon du liquide biologique. Cependant, ces procédés exigent beaucoup de temps et une grande technicité; dans le meilleur des cas, la durée nécessaire pour obtenir un résultat est d'environ 2 à 3 heures, ce qui n'est pas admissible en pratique.

Plus récemment, on a proposé un procédé microbiologique rapide pour détecter la présence d'antibiotiques dans un liquide biologique, en particulier dans le lait (cf. brevet américain n° 4.239.852).

Selon cette méthode, l'échantillon du liquide à examiner est incubé, d'une part avec des cellules ou parties de cellules d'un microorganisme très sensible aux antibiotiques, en particulier <u>Bacillus stearothermophilus</u> et d'autre part, avec un antibiotique marqué par un élément radioactif ou par une enzyme. Au cours de l'incubation, l'antibiotique éventuellement présent dans l'échantillon et l'antibiotique marqué entrent en compétition pour se fixer sur les sites récepteurs des cellules ou parties de cellules. Ensuite, on détermine la quantité d'antibiotique marqué qui a été fixée sur les cellules ou parties de cellules; ceci fournit une indication de la présence (ou non) d'antibiotiques, étant donné que la quantité d'antibiotique marqué fixée est inversément proportionnelle à la concentration d'antibiotique dans l'échantillon.

D'après l'auteur de ce brevet, ce procédé permettrait de détecter des concentrations en antibiotique aussi faibles que 0,01 U.I./ml, voire même de 0,001 U.I./ml dans le lait en un peu moins de 15 minutes.

Mais, l'inconvénient majeur de ce procédé réside dans le fait que, pour atteindre ce résultat, il faut obligatoirement avoir recours à un antibiotique marqué par un élément radioactif ($^{14}C$ ou $^{125}I$), qu'il faut doser à l'aide d'un appareil spécial, comme par exemple un compteur à scintillation. De plus, la manipulation de produits radioactifs, même en très faibles quantités, n'est pas totalement exempte de danger pour la personne qui effectue l'analyse.

Il est vrai que dans l'exemple 2 de ce brevet, on décrit une variante de ce procédé selon laquelle on utilise un antibiotique marqué par une enzyme et dans laquelle on détermine la quantité d'antibiotique marqué par une méthode colorimétrique visuelle. Mais, cette variante ne permet de détecter que s'il y a plus (ou moins) de 0,05 U.I./ml de pénicilline dans l'échantillon de lait. Ce procédé est donc nettement moins sensible et par conséquent beaucoup moins intéressant.

Il existe également dans le commerce des tests basés sur l'utilisation d'anticorps monoclonaux ou polyclonaux. En effet, il est connu que des tests peuvent être élaborés à partir des techniques classiques de diagnostic immunologique (ELISA, agglutination au latex, méthode radio-immunologique, etc.). A titre d'exemple de ce genre de test citons le SPOT TEST, commercialisé par la société ANGENICS (Etats-Unis).

Les désavantages de ce type de tests résident dans le fait qu'ils sont généralement très complexes et surtout qu'ils ne permettent de détecter qu'un nombre très limité d'antibiotiques. En effet, la haute spécificité des anticorps ne leur permet de reconnaître que les membres d'une famille structurellement liés à celui qui a été choisi pour l'immunisation. Ceci est particulièrement délicat pour la famille des β-lactames.

On connaît également un procédé enzymatique permettant de déterminer de faibles concentrations d'antibiotiques à noyau $\beta$-lactame dans le sérum humain et dans le lait (J-M. FRERE et al., Antimicrobial Agents and Chemotherapy, 18, (1980,n° 4), 506-510).

Ce procédé (désigné ci-après par "procédé de J-M. FRERE") est plus intéressant parce qu'il ne nécessite pas le recours à des produits radioactifs requérant des appareils de mesure sophistiqués, tout en étant d'une grande rapidité et d'une précision remarquable. Ce procédé est basé sur l'utilisation d'une enzyme spécifique, en l'occurrence la D-alanyl-D-alanine-carboxypeptidase exocellulaire soluble, qui est produite par Actinomadura R39 (précédemment dénommée Streptomyces R39). Dans la description qui va suivre, cette enzyme sera désignée par "enzyme R39". Cette enzyme possède une activité spécifique d'hydrolyse des groupements terminaux D-alanyl-D-alanine de divers peptides avec libération de la D-alanine terminale.

Une autre caractéristique importante de l'enzyme R39 réside dans le fait qu'elle réagit avec les antibiotiques à noyau $\beta$-lactame pour former très rapidement un complexe enzyme-antibiotique équimoléculaire, inactif et sensiblement irréversible.

Dans le procédé de J-M. FRERE, on met à profit ces propriétés de l'enzyme R39 pour déterminer de très faibles concentrations d'antibiotiques à noyau $\beta$-lactame. Ce procédé comporte trois stades essentiels.

Dans le premier stade, un volume déterminé d'un échantillon du liquide à examiner est incubé avec une quantité déterminée de l'enzyme R39. L'incubation est menée dans des conditions permettant à l'antibiotique $\beta$-lactame éventuellement présent dans l'échantillon de réagir avec l'enzyme pour former un complexe enzyme-antibiotique équimoléculaire, inactif et sensiblement irréversible.

Dans le deuxième stade, une quantité déterminée de substrat, par exemple, la $N^{alpha}$, $N^{epsilon}$-diacétyl-L-lysyl-D-alanyl-D-alanine, est incubée avec le produit obtenu au premier stade dans des conditions permettant l'hydrolyse du substrat par l'enzyme pour former une quantité de D-alanine correspondant à l'activité résiduelle de l'enzyme R39 qui n'a pas été complexée avec l'antibiotique dans le premier stade.

Dans le troisième stade, on détermine la quantité de D-alanine ainsi formée. On comprendra aisément que la quantité de D-alanine formée au deuxième stade dépend de l'activité résiduelle de l'enzyme et est donc inversément proportionnelle à la quantité d'antibiotique présente dans l'échantillon.

Dans le procédé de J-M. FRERE, on détermine cette quantité de D-alanine par une méthode enzymatique. Celle-ci repose sur deux réactions enzymatiques couplées. Dans la première réaction, la D-alanine est oxydée en acide pyruvique à l'aide d'une D-aminoacide-oxydase (en présence de son coenzyme, le flavine-adénine-dinucléotide); il se forme en même temps une quantité correspondante de peroxyde d'hydrogène à partir de l'oxygène de l'air. Dans la deuxième réaction, le peroxyde d'hydrogène formé est utilisé pour oxyder l'o-dianisidine à l'aide d'une peroxydase. Il se produit une coloration brune, dont l'intensité est fonction de la quantité de D-alanine.

Ceci permet donc de déterminer la quantité de D-alanine par une méthode colorimétrique, soit visuellement, soit par mesure de la densité optique au spectrophotomètre (lambda max = 460 nm).

En préparant une série d'échantillons d'une concentration connue en antibiotique et en appliquant ce procédé, on peut ainsi établir une courbe d'étalonnage qui relie le pourcentage d'activité enzymatique résiduelle de l'enzyme R39 à la concentration en antibiotique.

Pour obtenir une indication quantitative de la concentration en antibiotique d'un échantillon, on procède alors exactement de la même manière et on détermine la concentration en antibiotique en se reportant à cette courbe d'étalonnage.

Cette évaluation quantitative nécessite évidemment le recours à un spectrophotomètre.

Mais, pour déterminer si la concentration en antibiotique dépasse ou non une certaine valeur critique, il n'est pas nécessaire de recourir à un spectrophotomètre ou un autre appareil similaire de mesure.

Il suffit de connaître au préalable à quelle concentration critique en antibiotique l'activité de l'enzyme R39 est totalement supprimée ou, autrement dit, à quelle concentration en antibiotique, il ne se forme pas de D-alanine et, par conséquent, il ne se produit pas de coloration. Connaissant cette concentration critique, il est évident que l'on peut, par une simple inspection visuelle du résultat de la détermination, juger si oui ou non un échantillon donné contient une concentration en antibiotique moins grande ou plus grande que ladite concentration critique. On voit donc que par ce procédé on peut, sans disposer d'un appareil particulier, obtenir rapidement et de manière précise une indication de la concentration en antibiotique dans le lait.

En outre, ce procédé permet de déterminer des concentrations relativement faibles en antibiotique $\beta$-lactame dans le lait et dans le sérum humain. Ainsi par exemple, en partant d'échantillons de lait d'un volume d'environ 20 $\mu$l et en incubant ceux-ci avec 3 picomoles d'enzyme R39, il est possible de déterminer, quantitativement (en utilisant un spectrophotomètre), des concentrations en pénicilline aussi faibles que 0,02 U.I./ml de lait, et qualitativement, par la méthode visuelle décrite plus haut, des

concentrations de plus de 0,09 U.I./ml de lait en moins d'une heure.

Cependant, le procédé de J-M. FRERE présente divers inconvénients importants.

En effet, la sensibilité du procédé de J-M. FRERE, en particulier dans le cas de liquides biologiques (lait, salive, sérum, etc.) est insuffisante. Il est vrai que l'on pourrait augmenter cette sensibilité soit en diminuant la quantité d'enzyme R39 mise en oeuvre, mais il en résulterait une augmentation de la durée de réaction aussi bien au premier qu'au deuxième stade du procédé, soit en augmentant le volume de l'échantillon qu'il faut incuber avec l'enzyme R39, et dans ce cas, il faudrait augmenter le temps de réaction au premier stade du procédé.

Malheureusement, il n'est pas possible d'augmenter de cette manière la sensibilité du procédé de J-M. FRERE, en particulier dans le cas de liquides complexes d'origine biologique. On a constaté en effet qu'il est impossible de réaliser une détermination convenable lorsque le volume de l'échantillon de liquide biologique dépasse un certain volume critique, qui varie en fonction de la nature du liquide biologique. Ainsi, dans le cas du lait et du sérum, lorsque le volume de l'échantillon que l'on soumet à l'incubation dépasse un volume de ± 50 $\mu$l, on observe que la quantité de o-dianisidine oxydée formée est fortement réduite.

Enfin, dans le cas de l'urine, on ne détecte aucune activité enzymatique, même en utilisant des volumes d'échantillon aussi faibles que 10 $\mu$l. Il est donc impossible d'utiliser ce procédé pour la détermination d'antibiotiques dans l'urine.

On suppose que les liquides biologiques renferment des substances qui inhibent l'action des enzymes utilisées dans le procédé. Pratiquement, cela signifie que l'utilisation de grands volumes d'échantillons ne pourrait se faire dans les meilleurs cas qu'aux dépens d'une augmentation de la quantité des réactifs au moins proportionnelle à l'augmentation de volume.

Ceci n'est cependant pas à conseiller étant donné le coût élevé des enzymes mises en oeuvre, en particulier la D-aminoacide-oxydase et son cofacteur le flavine-adénine-dinucléotide.

Une version améliorée du procédé de J-M. FRERE est commercialisé sous la forme d'un test portant la marque "Penzym". Ce test est principalement utilisé comme test de triage pour le lait arrivant dans les laiteries.

Ce test est réalisé en deux stades; dans le premier stade, 50 $\mu$l de lait sont incubés avec 1,5 picomole d'enzyme R39 pendant 5 minutes à 47 °C. Ensuite, dans le second stade, tous les réactifs permettant de déterminer l'activité enzymatique résiduelle, en l'occurrence le substrat $N^{alpha}$, $N^{epsilon}$-diacétyl-L-lysyl-D-alanyl-D-alanine, la D-aminoacide-oxydase, le flavine-adénine-dinucléotide, la péroxydase et le réactif chromogène sont ajoutés au milieu d'incubation et l'ensemble est incubé pendant 15 minutes à 47 °C. Au terme de l'incubation, par comparaison avec une charte de couleurs, une couleur jaune dépourvue de rose indique la présence d'antibiotiques à noyau $\beta$-lactame à une concentration supérieure à 0,017 U.I./ml de lait. Par contre, la présence d'une coloration rose indique soit l'absence d'antibiotiques (coloration rose intense), soit la présence d'antibiotiques à des concentrations allant jusqu'à 0,017 U.I./ml, l'intensité de la couleur rose obtenue étant inversément proportionnelle à la concentration en antibiotique.

Grâce à ce test, on peut déterminer visuellement des concentrations en antibiotique dans le lait aussi faibles que 0,017 U.I./ml en 20 minutes.

Toutefois, même cette version optimalisée du procédé de J-M. FRERE présente des caractéristiques de sensibilité et de rapidité qui ne permettent pas son utilisation soit dans les pays où la législation impose des concentrations en antibiotique extrêmement faibles, par exemple de 0,004 U.I./ml, soit comme test ultra-rapide pour permettre au récolteur de lait d'effectuer des tests à la ferme en un temps de moins de 5 minutes.

L'augmentation de la sensibilité et la réduction du temps de réponse sont soumises aux mêmes contraintes que celles déjà signalées plus haut lors de la discussion du procédé de J-M. FRERE. En outre, ce test s'effectue sur de très faibles volumes d'échantillon et demande donc une certaine expérience dans la technique de microdosage pour pouvoir être effectué correctement.

Pour éviter tous les problèmes liés à l'utilisation d'un test se déroulant complètement en présence du liquide biologique, un procédé a également été proposé dans lequel l'enzyme R39 est immobilisée sur un support insoluble dans l'eau, en particulier sur une résine de poly(N,N-diméthylacrylamide). Ce procédé qui fait l'objet du brevet américain n° 4.546.076, comporte essentiellement les trois stades suivants:

(1) l'enzyme immobilisée sur son support est incubée en présence du liquide biologique;

(2) l'enzyme immobilisée est ensuite séparée du liquide biologique et lavée;

(3) l'activité résiduelle de l'enzyme R39 est déterminée par dosage colorimétrique de la D-alanine formée à partir du substrat peptidique grâce au système de réactifs comprenant une D-aminoacide-oxydase et son cofacteur, une peroxydase et un réactif chromogène, avec lecture visuelle du résultat ou à l'aide d'un spectrophotomètre.

Ce procédé présente plusieurs avantages:

- il n'y a plus aucune interférence du liquide biologique avec les enzymes utilisées pour le dosage de la D-alanine puisque le liquide biologique est déjà éliminé au stade (2) du procédé;
- on peut faire d'excellentes déterminations sur des volumes d'échantillon plus importants allant jusqu'à 5 ml; et
- la sensibilité est beaucoup plus grande vu que ce procédé permet de déterminer visuellement des concentrations en pénicilline G de plus de 0,002 U.I./ml de lait.

Toutefois, ce procédé présente aussi des inconvénients:

- comme tous les procédés qui nécessitent une étape de séparation, la reproductibilité des résultats est moins bonne que dans un procédé en phase homogène;
- quelle que soit la méthode de séparation mise en oeuvre, il en résulte inévitablement une augmentation de la durée et de la complexité du procédé;
- même si ce procédé permet d'obtenir par lecture visuelle une sensibilité élevée pouvant aller jusque 0,002 U.I./ml de pénicilline G, le temps nécessaire pour obtenir ce résultat est nettement trop long (au moins 30 minutes) pour que ce procédé puisse être utilisé pour le dépistage rapide au moment de la collecte du lait à la ferme; et - de plus, son coût est nettement plus élevé que celui d'un test pratiqué en phase homogène.

Un progrès technique et économique important serait donc de pouvoir disposer d'un procédé qui ne présente plus les divers inconvénients des procédés de l'état de la technique et en particulier du procédé de J-M. FRERE ou des procédés qui en sont dérivés tels que le test "Penzym" ou le procédé décrit dans le brevet américain n° 4.546.076. En d'autres termes, il faudrait notamment pouvoir mettre au point un procédé qui présente un ensemble de propriétés avantageuses, parce que

- on obtient très rapidement le résultat, par exemple en 5 minutes ou moins;
- il présente une sensibilité très élevée permettant son emploi même dans les pays où la législation est très sévère et impose des concentrations en antibiotique n'excédant pas 0,004 U.I./ml ou moins;
- il permet d'effectuer des déterminations sur des volumes d'échantillon importants de 1 ml ou plus, ce qui autoriserait son exécution aisée par du personnel non spécialisé;
- il est d'un faible coût et d'une très grande simplicité de mise en oeuvre.

Ces objectifs sont atteints par le procédé faisant l'objet de la présente invention qui se distingue par les caractéristiques essentielles suivantes:

(1) utilisation d'un substrat spécial de type thioester pour l'enzyme R39 de manière à former par hydrolyse un composé contenant un groupe SH libre qui peut être dosé par une méthode colorimétrique simple et peu coûteuse, sans nécessiter des enzymes dont l'action est perturbée par des composants du liquide biologique;

(2) conduite de l'hydrolyse du substrat par l'enzyme en présence d'un acide aminé de configuration D ou de glycine activant sensiblement cette hydrolyse.

La présente invention a donc pour objet un nouveau procédé enzymatique pour la détermination d'antibiotiques à noyau $\beta$-lactame dans un liquide biologique comprenant les stades suivants:

(1) l'incubation dudit liquide biologique avec la D-alanyl-D-alanine-carboxypeptidase soluble produite par Actinomadura R39, cette incubation étant menée dans des conditions permettant à l'antibiotique $\beta$-lactame éventuellement présent dans ledit liquide de réagir avec l'enzyme pour former un complexe enzyme-antibiotique équimoléculaire, inactif et sensiblement irréversible;

(2) l'incubation du mélange obtenu à la fin du stade (1) avec une solution de substrat dans des conditions permettant l'hydrolyse dudit substrat pour former un composé, dont la quantité est proportionnelle à l'activité enzymatique résiduelle;

(3) la détermination de la quantité du composé formée au stade (2);

(4) la comparaison de la valeur déterminée au stade (3) avec un étalon pour obtenir la concentration de l'antibiotique dans le liquide biologique,

qui est caractérisé en ce que l'incubation au stade (2) est effectuée avec un substrat qui est un thioester de formule générale

$$R_1\text{-}R_2\text{-}S\text{-}\underset{\displaystyle R_3}{\underset{|}{CH}}\text{-}COOH \qquad (I)$$

dans laquelle

$R_1$ représente le radical benzoyle, phénylacétyle ou N$^{alpha}$-acétyl-L-lysyle,

$R_2$ représente le radical glycyle ou D-alanyle, et

$R_3$ est un atome d'hydrogène ou un radical méthyle,

de manière à former par hydrolyse un acide 2-mercaptoalcanoïque de formule

$$\begin{array}{l} HS\text{-}CH\text{-}COOH \\ \quad\;\; | \\ \quad\;\; R_3 \end{array} \qquad (II)$$

dans laquelle $R_3$ a la signification donnée ci-dessus,

et en présence d'un acide aminé qui active l'hydrolyse du substrat par l'enzyme, ledit acide aminé étant choisi parmi un acide aminé de configuration D et la glycine.

Au cours de ses travaux de recherche dans ce domaine, la demanderesse a découvert que l'enzyme R39 possède une activité spécifique d'hydrolyse des liaisons thioester de certains composés possédant un groupement thioester terminal et plus particulièrement une activité d'hydrolyse vis-à-vis des thioesters répondant à la formule générale I mentionnée ci-dessus. Cette découverte est surprenante en soi car, à la connaissance de la demanderesse, on ne connaît pas dans la technique d'exemple d'une D-alanyl-D-alanine-carboxypeptidase qui fonctionne également comme une thioestérase. La demanderesse a tiré profit des avantages que procure cette propriété inattendue de l'enzyme R39 pour mettre au point un nouveau procédé enzymatique plus perfectionné pour la détermination des antibiotiques à noyau $\beta$-lactame dans les liquides biologiques.

A la différence du procédé de J-M. FRERE et des procédés analogues décrits plus haut, on utilise donc conformément à la présente invention, en tant que substrat pour l'enzyme R39, un thioester de formule générale I qui forme, par hydrolyse, un acide 2-mercaptoalcanoïque répondant à la formule II donnée ci-dessus. Ce nouveau substrat apporte de nombreux avantages tant sur le plan technologique que sur le plan économique. En effet, l'acide 2-mercaptoalcanoïque de formule II formé à partir de ce substrat (et dont la quantité est proportionnelle à l'activité résiduelle de l'enzyme R39) peut être dosé par une méthode colorimétrique simple qui ne fait plus appel à des enzymes telles que la D-aminoacide-oxydase et son coenzyme. Outre une réduction substantielle du prix de revient, on supprime donc un des inconvénients majeurs du procédé de J-M. FRERE, étant donné qu'il n'y a plus aucune interférence possible entre les composants du liquide biologique et les réactifs du dosage colorimétrique, ces derniers étant constitués par de simples réactifs chimiques exempts d'enzymes.

Par le procédé conforme à l'invention, on peut donc faire une détermination directement sur le liquide biologique tel quel; il n'est plus nécessaire d'enlever au préalable des échantillons soumis à la détermination, les substances qui peuvent perturber le dosage colorimétrique.

En outre, étant donné que la détermination de l'activité résiduelle de l'enzyme n'est plus influencée par des facteurs présents dans les liquides biologiques, il est possible aussi de travailler avec des volumes d'échantillon de liquide biologique pouvant aller jusque 10 ml. Le procédé conforme à l'invention permet par conséquent de travailler sur des volumes plus importants en le rendant par là même plus facile à exécuter par des personnes non spécialisées.

De surcroît, vu qu'il est possible de travailler sur de plus grands volumes, le procédé de l'invention permet d'obtenir une sensibilité beaucoup plus grande. Comme on le montrera ci-après dans les exemples de réalisation, le procédé de la présente invention permet de déterminer visuellement et avec facilité des quantités de l'ordre de 0,004 U.I. de pénicilline G par millilitre de lait en 15 minutes; on peut même, en se reportant à une charte de couleurs, parvenir à détecter des concentrations aussi faibles que 0,002 U.I./ml.

La demanderesse a en outre constaté avec surprise que l'addition d'un acide aminé de configuration D ou de glycine a pour effet d'augmenter considérablement la vitesse de la réaction d'hydrolyse du substrat de type thioester de formule I sous l'influence de l'enzyme. Ses travaux de recherche dans ce domaine ont montré que plusieurs acides aminés de configuration D de même que la glycine pouvaient jouer le rôle d'activateur de la réaction d'hydrolyse du thioester. Parmi les meilleurs activateurs on peut citer la D-alanine, la D-méthionine, la D-arginine, la D-phénylalanine, la D-serine, la D-histidine, la D-valine, le D-tryptophane et l'acide D-2-aminobutyrique.

Ainsi, par exemple, si l'activité de l'enzyme R39 est exprimée en unités par milligramme d'enzyme, une unité d'enzyme hydrolysant 1 micromole de substrat par minute, à 47 °C, on a trouvé que l'activité de l'enzyme R39 vis-à-vis de l'acide [(N-benzoyl-D-alanyl)thio]-acétique utilisé comme substrat, est égale à 8

unités en l'absence de D-alanine et à 320 unités en présence de D-alanine. En d'autres termes, en présence de cet activateur, la vitesse d'hydrolyse du substrat par l'enzyme R39 est 40 fois plus élevée.

Par contre, il est aussi apparu qu'aucun acide aminé ayant la configuration L n'est capable d'activer l'hydrolyse du substrat.

C'est pourquoi, conformément à l'invention, il est essentiel que l'hydrolyse du substrat de type thioester, au stade (2), soit effectuée en présence d'un acide aminé de configuration D qui active l'hydrolyse dudit substrat par l'enzyme R39. Cette mesure permet en effet un gain de temps considérable pour réaliser le procédé. C'est ainsi que l'on parvient grâce au procédé conforme à la présente invention à déterminer facilement une concentration en pénicilline G de 0,016 U.I. par millilitre de lait en un temps de 5 minutes. A titre de comparaison rappelons que le test "Penzym" requiert environ 15 minutes pour obtenir le même résultat. Contrairement aux procédés de l'état de la technique, le procédé conforme à l'invention est donc particulièrement attrayant pour le dépistage rapide au moment de la collecte du lait à la ferme, d'autant plus que sa mise en oeuvre est simple et ne nécessite pas un appareillage sophistiqué.

En outre, on a constaté que le procédé conforme à la présente invention peut être appliqué avec succès, non seulement pour la détermination d'antibiotiques dans le lait, mais également dans d'autres liquides biologiques comme par exemple le sérum, l'urine, la salive, les extraits de viande, des liquides de fermentation, des solutions aqueuses tamponnées.

L'avantage primordial du procédé de la présente invention est donc qu'il permet, soit de détecter en un temps très court (de 5 minutes) des concentrations en antibiotique $\beta$-lactame de l'ordre d'environ 0,016 U.I./ml, soit en un temps un peu plus long (de 15 minutes) de très faibles concentrations en antibiotique $\beta$-lactame de l'ordre de 0,004 U.I./ml ou moins, sans devoir recourir à un appareil particulier d'analyse et sans devoir faire appel à une technologie complexe qui nécessite un procédé de séparation.

Les antibiotiques, dont on peut déterminer la concentration en utilisant le procédé conforme à l'invention, appartiennent au groupe d'antibiotiques qui sont caractérisés par la présence d'un noyau $\beta$-lactame dans leur molécule, c'est-à-dire en principe toutes les pénicillines et les céphalosporines. A titre d'exemple de pénicillines, on peut citer la benzylpénicilline (pénicilline G), l'ampicilline, la phénoxyméthylpénicilline, la carbénicilline, la méthicilline, l'oxacilline, la cloxacilline. A titre d'exemple de céphalosporines, on peut citer la céphalosporine C, la céfaloglycine, la céfalothine, la céfalexine, la céfapirine.

Des résultats particulièrement favorables ont été obtenus avec la pénicilline G.

Au stade (1) du procédé conforme à l'invention, un volume déterminé d'un échantillon du liquide biologique est incubé avec une quantité déterminée de l'enzyme R39.

Comme il a été expliqué plus haut, il est possible de travailler avec de très grands volumes d'échantillons. Pour une quantité donnée d'enzyme R39, en augmentant le volume de l'échantillon, on augmente parallèlement la sensibilité du procédé. Par exemple, en doublant le volume de l'échantillon, la sensibilité est doublée, en triplant le volume de l'échantillon, la sensibilité est triplée, etc. On peut donc choisir le volume de l'échantillon en fonction de la sensibilité désirée. Dans le cas du lait par exemple, on pourra adapter la sensibilité du procédé aux normes fixées par la législation du pays où il est utilisé, ou encore aux exigences de l'industrie laitière.

On peut également obtenir le même effet pour un volume donné de liquide biologique en diminuant la quantité d'enzyme R39 (cf. l'exemple 2 ci-après). Toutefois, dans ce cas, il faut augmenter proportionnellement non seulement la durée du stade (1), mais également la durée du stade (2) du procédé.

En pratique, le choix des quantités d'enzyme R39 et de liquide biologique à utiliser dépendra, d'une part, de la façon dont le procédé sera mis en oeuvre et, d'autre part, de la sensibilité ou de la rapidité que l'on désire obtenir.

Ainsi par exemple, si l'on désire utiliser le procédé conforme à l'invention pour déterminer la contamination du lait à la ferme, on préférera travailler avec de grands volumes d'échantillon afin de rendre la manipulation plus aisée. On choisira de préférence des volumes compris entre 1 et 10 ml. Pour effectuer ce procédé dans un laboratoire équipé de matériel de microdosage, des volumes d'échantillon compris entre 50 $\mu$l et 1 ml conviennent parfaitement.

Par ailleurs, le choix des quantités d'enzyme et de liquide biologique à mettre en oeuvre dépendra essentiellement de la sensibilité recherchée.

Ainsi par exemple, en laboratoire où de petits volumes peuvent être utilisés et où l'on désire obtenir une sensibilité de 0,01 U.I./ml, on choisira une quantité d'enzyme de l'ordre de 1 picomole et un volume de liquide biologique de l'ordre de 50 $\mu$l. Si l'on désire obtenir une sensibilité respectivement de 0,005 et 0,0025 U.I./ml, on portera le volume de liquide biologique respectivement à 100 $\mu$l et à 200 $\mu$l, sans changer la quantité d'enzyme.

L'excellente sensibilité, la rapidité et la précision du procédé conforme à l'invention résultent des caractéristiques particulières de l'enzyme R39 d'une part et, d'autre part, de la méthode utilisée pour la

détermination de l'activité résiduelle de cette enzyme R39.

En effet, l'enzyme R39 est caractérisée par:

- la formation extrêmement rapide d'un complexe enzyme-antibiotique équimoléculaire inactif,
- la stabilité extraordinaire dudit complexe parce que, une fois formé, il ne se décompose que fort lentement. A titre d'exemple, le temps de demi-vie du complexe formé entre l'enzyme R39 et la pénicilline G est d'environ 70 heures à 37 ° C,
- une excellente activité enzymatique se traduisant par une hydrolyse très rapide du groupement terminal du substrat de type thioester de formule I sous l'influence d'un activateur spécifique.

Grâce à ces trois caractéristiques, l'enzyme R39 occupe une position privilégiée par rapport aux D-alanyl-D-alanine-carboxypeptidases identifiées jusqu'à présent. En effet, étant donné que le temps de décomposition du complexe enzyme-antibiotique est infiniment plus grand que la durée totale qu'il faut respectivement pour la formation du complexe et pour la mesure de l'activité enzymatique résiduelle, il n'est pas à craindre que les résultats de la détermination soient faussés par une remise en liberté d'enzyme active à la suite d'une décomposition prématurée du complexe enzyme-antibiotique.

Or, lorsqu'on examine les D-alanyl-D-alanine-carboxypeptidases que l'on connaît aujourd'hui, hormis l'enzyme R39, il n'y en a aucune autre qui réponde parfaitement à ces conditions; en effet, c'est soit la vitesse de formation du complexe qui est une dizaine de fois plus faible, soit la stabilité du complexe antibiotique-enzyme qui est absolument insuffisante, soit encore la vitesse d'hydrolyse du substrat qui est beaucoup trop lente (c'est le cas notamment de toutes les carboxypeptidases endocellulaires liées à la membrane bactérienne).

L'enzyme R39 est la D-alanyl-D-alanine-carboxypeptidase exocellulaire soluble spécifique qui est excrétée par Actinomadura R39, lorsqu'on cultive ce microorganisme (déposé le 10 juillet 1981 à l'Institut Pasteur à Paris, sous le n° I-127) dans un milieu de culture approprié.

Pour la mise en oeuvre du procédé de l'invention, cette enzyme doit évidemment être substantiellement pure. Sa préparation et sa purification peuvent être effectuées suivant les méthodes décrites dans la littérature (voir à ce sujet l'article de J-M. FRERE & al., Biochem.J.143,(1974),233-240, intitulé "Molecular Weight, Aminoacid Composition and Physicochemical Properties of the Exocellular DD-Carboxypeptidase-Transpeptidase of Streptomyces R39). Toutefois, cette enzyme, à l'état pur, est maintenant disponible dans le commerce; on peut se la procurer auprès de la société UCB-BIOPRODUCTS S.A. (Belgique).

L'enzyme R39 possède une excellente stabilité; elle supporte des températures élevées pouvant aller jusque 60 ° C. C'est pourquoi, l'incubation du liquide biologique avec l'enzyme R39 peut être effectuée sans inconvénients dans un intervalle de température compris entre 20 et 50 ° C. De préférence, cette température est voisine de 47 ° C. En effet, dans ces conditions, la durée de l'incubation, qui est étroitement liée au temps nécessaire à la formation du complexe enzyme-antibiotique équimoléculaire inactif, est très courte. Une augmentation de la température de l'incubation aura pour effet une diminution de sa durée et inversément. Il est donc possible de raccourcir la durée du procédé en augmentant la température.

Au stade (2) du procédé conforme à la présente invention, le mélange obtenu au terme du stade (1) est incubé avec une quantité déterminée du substrat de type thioester de formule I, en solution, en présence de glycine ou d'un acide aminé de configuration D, qui active l'hydrolyse de ce substrat par l'enzyme. Au cours de ce stade, le fraction de l'enzyme qui n'a pas été consommée dans la formation du complexe enzyme-antibiotique au stade (1) du procédé est utilisée pour effectuer l'hydrolyse du substrat thioester de formule I. Cette réaction d'hydrolyse produira une quantité d'un acide 2-mercaptoalcanoïque de formule II qui est proportionnelle à l'activité résiduelle de l'enzyme R39.

Les thioesters de formule I sont des composés nouveaux, à l'exception de l'acide [(N-benzoyl-glycyl)-thio]-acétique, qui est un composé connu (cf. brevet américain n° 2.824.863).

Les thioesters de formule I peuvent être préparés selon un procédé conventionnel qui ne fait intervenir que des réactions connues en elle-mêmes. En règle générale, on condense dans un solvant inerte tel que le chloroforme, le dichlorométhane, l'acétate d'éthyle ou le diméthylformamide, un acide de formule III, préalablement activé, par exemple sous la forme d'anhydrides mixtes ou encore d'esters actifs, avec un acide 2-mercaptoalcanoïque de formule II, selon le schéma réactionnel

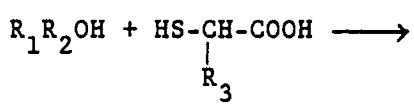

(I)

(III) (II)

dans lequel $R_1$, $R_2$ et $R_3$ ont la même signification que précédemment.

Comme exemples de thioesters de formule I, que l'on peut utiliser en tant que substrat, on citera l'acide [(N-benzoyl-D-alanyl)thio]-acétique, l'acide 2-[(N-benzoyl-D-alanyl)thio]-propionique, l'acide [(N-phénylacétyl-D-alanyl)thio]-acétique, l'acide [($N^{alpha}$-acétyl-L-lysyl-D-alanyl)thio]-acétique. Cependant, l'acide [(N-benzoyl-D-alanyl)thio]-acétique procure les meilleurs résultats.

En tant qu'exemples non limitatifs d'acides aminés de configuration D que l'on peut associer en tant qu'activateurs aux substrats mentionnés ci-dessus, on citera la D-alanine, la D-méthionine, la D-arginine, la D-phénylalanine, la D-serine, la D-histidine, la D-valine, le D-tryptophane et l'acide D-2-aminobutyrique.

Selon une forme particulièrement préférée de l'invention, la D-alanine est associée à l'acide [(N-benzoyl-D-alanyl)thio]-acétique, utilisé comme substrat.

Les quantités de substrat et d'activateur à mettre en oeuvre ne sont pas critiques pour autant que l'on opère dans des conditions de saturation de l'enzyme par le substrat.

Les conditions opératoires à observer au cours de ce stade sont sensiblement les mêmes que celles indiquées plus haut pour le stade (1). L'incubation peut être effectuée dans un intervalle de température compris entre 20 et 50 °C, de préférence à une température voisine de 47 °C. La durée de l'incubation avec la fraction de l'enzyme R39 non inactivée doit être au moins suffisante pour former une quantité mesurable d'acide 2-mercaptoalcanoïque de formule II. Pour une température d'incubation de 47 °C, cette durée peut varier entre quelques secondes et 10 minutes selon la quantité d'enzyme présente et le volume de l'échantillon. Cette durée peut être diminuée en augmentant la température de l'incubation ou, au contraire augmentée en diminuant la température de l'incubation. On a donc également intérêt à augmenter la température de cette incubation dans tous les cas où la rapidité de la détermination est un facteur important.

Afin de conserver une activité enzymatique optimale, le milieu d'incubation devra, de préférence, présenter une valeur de pH comprise entre 7 et 8,5. Habituellement, on maintient une valeur de pH voisine de 8,0 en effectuant l'incubation au sein d'un tampon approprié.

Au stade (3) du procédé conforme à la présente invention, on détermine la quantité d'acide 2-mercaptoalcanoïque de formule II qui a été formée au stade (2).

Le dosage de l'acide 2-mercaptoalcanoïque de formule II peut être effectué par n'importe quelle méthode connue en soi. Il importe seulement que la méthode soit rapide, peu onéreuse et qu'elle permette de déterminer spécifiquement l'acide 2-mercaptoalcanoïque de formule II à l'exclusion de tous les autres produits qui sont présents dans le liquide à examiner.

C'est pourquoi, on utilise de préférence un dosage colorimétrique à l'aide d'un réactif qui produit une coloration par réaction avec le groupe SH libre de l'acide 2-mercaptoalcanoïque.

Ce réactif chromogène pourra être choisi parmi les réactifs chimiques habituellement utilisés pour ce genre de réaction, comme par exemple l'acide 5,5'-dithiobis(2-nitrobenzoïque), les produits de condensation de la 4,4'-dithiobis(phénylamine) avec des benzaldéhydes, le système phénanthroline-$Fe^{+++}$.

Un réactif chromogène préféré est l'acide 5,5'-dithiobis(2-nitrobenzoïque), appelé également réactif d'Ellman, qui produit une coloration jaune basée sur l'acide 2-nitro-5-mercaptobenzoïque formé par une réaction d'échange avec le groupe SH de l'acide 2-mercaptoalcanoïque; l'intensité de cette coloration est donc directement fonction de la quantité d'acide 2-mercaptoalcanoïque.

Toutefois, pour certaines applications, il peut s'avérer préférable d'utiliser la coloration rouge qui est produite par la réaction de la phénanthroline avec des groupes SH libres en présence de cations $Fe^{+++}$.

La quantité de réactif chromogène à mettre en oeuvre sera fonction de la quantité d'acide 2-mercaptoalcanoïque formé dans l'application particulière. On préfère travailler dans des conditions où la quantité molaire de réactif chromogène est en léger excès par rapport à la quantité molaire maximum d'acide 2-mercaptoalcanoïque susceptible d'être formé au stade (2) du procédé.

On notera que l'enzyme R39 doit être nécessairement présente dès le début du stade (1) du procédé; de même, le substrat et l'activateur doivent être nécessairement présents au début du stade (2). Toutefois, il est possible d'ajouter l'activateur au début du stade (1), de même qu'il est possible d'ajouter le réactif chromogène au début soit de la première, soit de la deuxième étape, ou encore seulement à la fin du stade (2). Selon une forme de réalisation préférée, au stade (1) du procédé on incubera la solution de l'enzyme R39 avec l'échantillon de lait en présence de l'activateur, au stade (2) on ajoutera le substrat et le réactif chromogène et on exécute les stades (2) et (3) simultanément dans des conditions sensiblement identiques à celles indiquées plus haut pour le stade (2).

Au stade (4) du procédé, la valeur déterminée au stade (3) est comparée avec un étalon pour obtenir la concentration de l'antibiotique dans le liquide biologique.

La détermination quantitative de la concentration en antibiotique peut être effectuée selon la méthode qui suit.

Tout d'abord, on prépare une série d'échantillons du liquide biologique renfermant une concentration connue en antibiotique $\beta$-lactame.

Cette série contiendra, en plus d'un certain nombre d'échantillons contenant une concentration croissante en antibiotique, deux échantillons du liquide biologique exempts d'antibiotiques.

Ensuite, on traite tous ces échantillons de manière rigoureusement identique en suivant les stades (1), (2) et (3) du procédé conforme à la présente invention.

Pour l'un des échantillons exempt d'antibiotique, on remplace toutefois la solution d'enzyme utilisée au stade (1) par un volume identique d'eau. Dans ce cas particulier, on obtiendra donc, au terme du stade (3), une solution qui contient tous les réactifs à l'exception de l'enzyme R39. Etant donné l'absence de l'enzyme, il ne se formera donc pas d'acide 2-mercaptoalcanoïque de formule II au stade (2) et par voie de conséquence le réactif chromogène, en l'occurrence l'acide 5,5'-dithiobis(2-nitrobenzoïque) ne donnera aucune coloration. Cet échantillon sera désigné ci-après par "échantillon blanc".

L'autre échantillon exempt d'antibiotique produira au contraire une coloration jaune prononcée. En effet, comme l'échantillon est exempt d'antibiotique, l'enzyme R39 n'est pas inactivée au stade (1) et il se formera une quantité maximum d'acide 2-mercaptoalcanoïque de formule II (correspondant à l'activité totale de l'enzyme R39 mise en oeuvre) et donc une quantité maximum d'acide 5-mercapto-2-nitrobenzoïque, et par voie de conséquence, il se produira une coloration jaune prononcée. Cet échantillon sera désigné ci-après par "échantillon témoin".

On comprendra aussi que lorsque les échantillons contiennent une quantité molaire d'antibiotique qui est inférieure à la quantité molaire d'enzyme R39 mise en oeuvre, une fraction seulement de cette enzyme sera inactivée par l'antibiotique au stade (1); dans ces cas, il se formera donc une quantité d'acide 2-mercaptoalcanoïque de formule II qui correspondra à l'activité résiduelle de la fraction de l'enzyme qui n'a pas été inactivée par l'antibiotique et par conséquent aussi une quantité correspondante d'acide 5-mercapto-2-nitrobenzoïque. Dans ces cas, il se produira donc également une coloration jaune, mais d'une intensité inférieure à celle observée avec l'échantillon témoin.

Enfin, lorsque les échantillons contiennent une quantité molaire d'antibiotique qui est égale ou supérieure à la quantité molaire d'enzyme R39 mise en oeuvre, l'enzyme sera complètement inactivée par l'antibiotique au cours du stade (1); il ne se formera donc pas d'acide 2-mercaptoalcanoïque de formule II et le réactif chromogène restera inchangé. Dans ces cas, on obtiendra donc une coloration identique à celle observée avec l'échantillon blanc.

Pour obtenir une détermination précise, on mesure au spectrophotomètre la densité optique des colorations obtenues respectivement avec tous les échantillons y compris les échantillons blanc et témoin.

Comme on trouve aussi pour l'échantillon blanc une certaine valeur de densité optique, il est nécessaire de déduire cette valeur de celles trouvées respectivement pour l'échantillon témoin et pour les autres échantillons.

A partir des valeurs de densité optique ainsi obtenues, on calcule le pourcentage d'activité résiduelle (de l'enzyme R39) pour chaque échantillon. Ce pourcentage est égal au rapport, multiplié par 100, entre les valeurs de densité optique trouvées respectivement pour un échantillon déterminé et pour l'échantillon témoin.

On établit ensuite un graphique, portant en abscisses, les concentrations en antibiotique, et en ordonnées, les pourcentages d'activité résiduelle de l'enzyme R39.

On obtient une droite qui coupe respectivement l'ordonnée en un point correspondant à l'échantillon témoin (100% d'activité enzymatique résiduelle) et l'abscisse en un point correspondant à l'échantillon contenant une quantité d'antibiotique égale à la quantité molaire d'enzyme R39 mise en oeuvre (0% d'activité enzymatique résiduelle).

Le graphique ainsi obtenu constitue un "étalon" qui permet de déterminer une concentration inconnue en antibiotique $\beta$-lactame dans un échantillon du liquide biologique ayant servi à son établissement. A cet effet, on traite cet échantillon de manière rigoureusement identique en suivant les stades (1), (2) et (3) du procédé conforme à l'invention; on mesure au spectrophotomètre la densité optique de la coloration obtenue; on en retranche la valeur de la densité optique trouvée pour l'échantillon blanc; on calcule le pourcentage d'activité enzymatique résiduelle de la manière indiquée plus haut et on obtient la concentration en antibiotique de l'échantillon en se reportant à l'étalon.

On peut ainsi déterminer de manière quantitative des concentrations en antibiotique aussi faibles que 0,002 U.I./ml dans un liquide biologique en 15 minutes.

Cependant cette méthode requiert au préalable dans certains cas la clarification du liquide biologique permettant l'emploi d'un spectrophotomètre et doit donc, en principe, être exécutée dans un laboratoire. Or, si l'on envisage uniquement de déterminer si la concentration en antibiotique dépasse ou non un certain seuil (par exemple dans le cas du lait une concentration maximum imposée par les normes légales), il n'est

pas nécessaire d'avoir recours à un spectrophotomètre.

Ceci nécessite cependant au préalable quelques explications.

Comme on l'a vu plus haut, la courbe d'étalonnage coupe l'abscisse en un point correspondant à un échantillon contenant une quantité d'antibiotique égale à la quantité molaire d'enzyme R39 mise en oeuvre. A cette concentration en antibiotique critique, le pourcentage d'activité enzymatique résiduelle est de zéro %, parce que au terme du stade (3) du procédé conforme à l'invention, on obtient une coloration identique à celle obtenue avec l'échantillon blanc.

Au-dessus de cette concentration critique, on obtient également une coloration identique à celle de l'échantillon blanc, parce que le pourcentage d'activité enzymatique résiduelle est toujours de zéro %. Par contre, au-dessous de cette concentration critique, on obtient une coloration jaune parce qu'il subsiste un certain pourcentage d'activité enzymatique résiduelle.

Par conséquent, en se basant simplement sur la coloration observée au terme du stade (3) du procédé, on peut évaluer directement dans un échantillon si la concentration en antibiotique dépasse ou non ladite concentration critique.

Il suffit donc de connaître d'avance ladite concentration critique, pour que l'on puisse ensuite, sans avoir recours à un spectrophotomètre, déterminer rapidement si un échantillon contient une concentration en antibiotique $\beta$-lactame qui dépasse (ou non) cette concentration critique. A cet effet, on traite cet échantillon de manière identique suivant les stades (1), (2) et (3) du procédé conforme à l'invention et ensuite on observe simplement la coloration obtenue; si celle-ci correspond à celle de l'échantillon blanc, la concentration en antibiotique sera au moins égale à la concentration critique; si par contre, elle est jaune, la concentration en antibiotique sera inférieure à la concentration critique.

On peut ainsi déterminer, visuellement et avec certitude, si des échantillons renferment plus ou moins de 0,004 U.I. d'antibiotique par ml de liquide biologique et ce en un temps de 15 minutes.

De plus, par l'utilisation d'une charte de couleurs reprenant la variation de l'intensité de la coloration jaune obtenue en fonction de la concentration en antibiotique, il sera possible de déterminer semi-quantitativement des concentrations intermédiaires entre 0 U.I./ml et la concentration critique. Ainsi dans une application où la concentration critique est de 0,004 U.I./ml, il sera possible de déterminer à tout le moins sans difficulté particulière une concentration de 0,002 U.I./ml.

Cette méthode avec ou sans charte de couleurs convient donc parfaitement pour l'examen en série d'échantillons de lait, même en dehors d'un laboratoire, par exemple, sur place à la ferme, par du personnel non spécialisé.

La méthode de détermination quantitative et qualitative de la concentration en antibiotique conforme à la présente invention, vient d'être expliquée en détail en se référant plus particulièrement au changement de couleur produit par l'acide 5,5'-dithiobis(2-nitrobenzoïque). Cependant, l'homme du métier comprendra aisément qu'en se servant dans cette méthode d'autres réactifs chromogènes, seule la couleur observée sera différente.

Un autre objet de la présente invention est de procurer une trousse d'essai utilisable pour la mise en oeuvre du procédé conforme à l'invention, c'est-à-dire pouvant servir à la détermination d'antibiotiques à noyau $\beta$-lactame dans un liquide biologique.

Cette trousse comprend notamment:

(1) une quantité déterminée de D-alanyl-D-alanine-carboxypeptidase soluble produite par <u>Actinomodura R39</u> (enzyme R39);

(2) une quantité déterminée d'un substrat qui est un thioester de formule générale

$$R_1-R_2-S-\underset{\displaystyle R_3}{\underset{|}{C}}H-COOH \qquad (I)$$

dans laquelle

- $R_1$ représente le radical benzoyle, phénylacétyle ou $N^{alpha}$-acétyl-L-lysyle,
- $R_2$ représente le radical glycyle ou D-alanyle, et
- $R_3$ est un atome d'hydrogène ou un radical méthyle,

(3) une quantité déterminée d'un acide aminé de configuration D ou de glycine;

(4) un réactif permettant la détermination d'un acide 2-mercaptoalcanoïque de formule

$$HS-\underset{R_3}{\underset{|}{CH}}-COOH \qquad (II)$$

dans laquelle $R_3$ à la signification donnée ci-dessus;

(5) éventuellement un étalon, par rapport auquel les résultats d'essais réalisés avec les réactifs (1), (2), (3) et (4) peuvent être comparés.

Selon une forme de réalisation préférée, le substrat est l'acide [(N-benzoyl-D-alanyl)thio]-acétique, l'acide aminé de configuration D est la D-alanine et le réactif (4) est l'acide 5,5'-dithiobis(2-nitrobenzoïque).

Selon une forme de réalisation particulièrement préférée, le substrat et le réactif permettant la détermination de l'acide 2-mercaptoalcanoïque sont réunis sous la forme d'un comprimé en association avec les excipients appropriés généralement utilisés pour former un comprimé.

En tant qu'étalon, on peut éventuellement incorporer dans la trousse un graphique établissant la relation entre les concentrations en antibiotique et les pourcentages d'activité résiduelle de l'enzyme R39. On peut ainsi réaliser des déterminations quantitatives ainsi qu'il a été expliqué plus haut. Ce graphique n'est toutefois pas indispensable. En effet, si la trousse doit servir uniquement pour déterminer si la concentration en antibiotique dépasse ou non une certaine limite, il suffit d'indiquer dans le mode d'emploi à quelle concentration en antibiotique on observe un virage de la coloration, après traitement de l'échantillon selon le procédé conforme à l'invention.

Les exemples suivants illustrent la présente invention, sans toutefois la limiter.

Exemple 1.

Cet exemple montre que le procédé conforme à l'invention permet une détermination très rapide de faibles concentrations de pénicilline G dans le lait.

On prépare une série d'échantillons de 50 µl de lait renfermant des concentrations connues en pénicilline G, ainsi que deux échantillons (blanc et témoin) de 50 µl de lait, exempts de pénicilline G.

A chaque échantillon (échantillon témoin + échantillons contenant de la pénicilline G), on ajoute 1,5 picomole d'enzyme R39 dissous dans 10 µl de tampon Hepes 0,5 M (pH = 8,0) contenant du NaCl 0,5 M et du $MgCl_2$ 0,25 M et 20 µl d'une solution aqueuse contenant 50 mg/ml de D-alanine. Dans l'échantillon blanc, l'enzyme R39 dissoute dans le tampon Hepes est remplacée par 10 µl de tampon Hepes 0,5 M (pH = 8,0) contenant du NaCl 0,5 M et du $MgCl_2$ 0,25 M (Hepes = acide 4-hydroxyéthyl-1-pipérazineéthanesulfonique). On incube le mélange à 47 °C pendant 4 minutes.

Ensuite, on ajoute 5 µl d'une solution aqueuse contenant 5 mg/ml d'acide [(N-benzoyl-D-alanyl)thio]-acétique et 10 µl d'une solution de tampon phosphate (pH = 8,0, $KH_2PO_4$ + $K_2HPO_4$) contenant 2 mg/ml d'acide 5,5'-dithiobis(2-nitrobenzoïque) et on incube le mélange à 47 °C pendant 1 minute.

On observe ensuite les colorations obtenues avec les divers échantillons.

Les résultats obtenus sont reproduits au tableau I.

TABLEAU I

| Echantillon | Concentration en pénicilline G (en U.I./ml) | Coloration |
|---|---|---|
| témoin | 0 | jaune très intense |
| 1 | 0,004 | jaune intense |
| 2 | 0,008 | jaune moyen |
| 3 | 0,012 | jaune pâle |
| 4 | 0,016 | blanche |
| 5 | 0,020 | blanche |
| 6 | 0,025 | blanche |
| blanc | 0 | blanche |

Au tableau I on voit que pour une concentration en pénicilline G égale ou supérieure à 0,016 U.I./ml, on obtient une coloration blanche identique à celle obtenue pour l'échantillon blanc, tandis que en dessous de

cette concentration, il se développe une coloration jaune qui est de plus en plus intense en fonction de la diminution de la concentration en pénicilline G, jusqu'à atteindre une coloration jaune très intense correspondant à une concentration nulle en pénicilline G (échantillon témoin).

Il apparaît clairement que le procédé conforme à l'invention permet, en 5 minutes de temps, de déterminer visuellement si un échantillon de lait contient une concentration en pénicilline G de 0,016 U.I./ml ou plus, ce qui rend le procédé particulièrement attrayant pour un triage rapide du lait, soit à la laiterie, soit à la ferme.

Exemple 2.

On montre dans cet exemple que le procédé conforme à l'invention est très sensible.

Le procédé est exactement le même qu'à l'exemple 1, sauf que:

- on ajoute aux échantillons 0,4 picomole d'enzyme R39 (au lieu de 1,5 picomole);
- la première incubation dure 10 minutes (au lieu de 4 minutes) et la seconde incubation dure 5 minutes (au lieu de 1 minute).

Les résultats obtenus sont reproduits au tableau II

TABLEAU II

| Echantillon | Concentration en pénicilline G (en U.I./ml) | Coloration |
|---|---|---|
| témoin | 0 | jaune très intense |
| 1 | 0,001 | jaune intense |
| 2 | 0,002 | jaune moyen |
| 3 | 0,003 | jaune pâle |
| 4 | 0,004 | blanche |
| 5 | 0,005 | blanche |
| 6 | 0,008 | blanche |
| 7 | 0,010 | blanche |
| blanc | 0 | blanche |

Ce tableau montre qu'en appliquant le procédé conforme à l'invention, il est possible de détecter visuellement dans le lait des concentrations en pénicilline G au moins égales à 0,004 U.I. par ml de lait en 15 minutes. De plus, en se reportant à une charte de couleurs, il est même possible de détecter des concentrations plus faibles telles que 0,002 U.I./ml. Ce procédé permet donc de déterminer si le lait contient une concentration en antibiotique qui dépasse (ou non) les normes légales, même dans les pays où ces normes sont relativement sévères.

Exemple 3.

Cet exemple montre qu'il est possible d'utiliser le procédé de l'invention pour mesurer des concentrations en antibiotique dans des volumes d'échantillon plus importants.

Le procédé est réalisé de la même manière qu'à l'exemple 1, excepté que:

- les échantillons contiennent 3 ml de lait (au lieu de 50 µl);
- on ajoute à chaque échantillon, 24 picomoles d'enzyme R39 dissous dans 150 µl de tampon Hepes 0,5 M (pH = 8,0) (au lieu de 1,5 picomole) et 600 µl d'une solution aqueuse de D-alanine contenant 100 mg/ml de D-alanine;
- la première incubation est menée pendant 10 minutes (au lieu de 4 minutes);
- on ajoute ensuite 120 µl de la solution aqueuse d'acide [(N-benzoyl-D-alanyl)thio]-acétique (au lieu de 5 µl) et 120 µl d'une solution de tampon phosphate (pH = 8,0) contenant 5 mg/ml d'acide 5,5'-dithiobis(2-nitrobenzoïque);
- la seconde incubation dure 5 minutes (au lieu de 1 minute).

Les résultats obtenus sont repris au tableau III:

TABLEAU III

| Echantillon | Concentration en pénicilline G (en U.I./ml) | Coloration |
|---|---|---|
| témoin | 0 | jaune très intense |
| 1 | 0,002 | jaune moyen |
| 2 | 0,003 | jaune pâle |
| 3 | 0,004 | blanche |
| 4 | 0,006 | blanche |
| blanc | 0 | blanche |

Ce tableau montre que le procédé de l'invention permet de détecter visuellement sur des volumes importants d'échantillon (3 ml) la présence de 0,004 U.I. de pénicilline G par ml de lait en 15 minutes. Il peut donc être exécuté très facilement par des personnes non spécialisées.

On obtient les mêmes résultats lorsqu'on utilise le procédé de l'invention pour mesurer des concentrations en pénicilline G dans des volumes d'échantillon de 1 ml de lait.

Exemple 4.

Cet exemple montre qu'il est possible d'utiliser le procédé de l'invention avec d'autres substrats que l'acide [(N-benzoyl-D-alanyl)-thio]-acétique.

Le procédé est réalisé de la même manière qu'à l'exemple 3. Toutefois, après la première incubation, on ajoute 120 µl d'une solution aqueuse contenant 5 mg/ml d'acide [(N-phénylacétyl-D-alanyl)thio]-acétique.

Les résultats obtenus sont repris dans le tableau IV:

TABLEAU IV

| Echantillon | Concentration en pénicilline G (en U.I./ml) | Coloration |
|---|---|---|
| témoin | 0 | jaune très intense |
| 1 | 0,003 | jaune pâle |
| 2 | 0,006 | blanche |
| blanc | 0 | blanche |

Ce tableau montre que le procédé selon l'invention peut être mis en oeuvre avec succès en utilisant l'acide [(N-phénylacétyl-D-alanyl)thio]-acétique comme substrat.

Exemple 5.

Cet exemple montre que le procédé de l'invention peut être mis en oeuvre en utilisant, comme activateurs de l'hydrolyse du substrat par l'enzyme, divers acides aminés de configuration D ou la glycine.

Le procédé est réalisé de la même manière qu'à l'exemple 3. Toutefois, la solution aqueuse contenant 100 mg/ml de D-alanine (essai I) est remplacée respectivement par une solution aqueuse contenant 100 mg/ml de D-phénylalanine (essai II), de D-sérine (essai III), de D-histidine (essai IV), de D-méthionine (essai V), d'acide D-2-aminobutyrique (essai VI) ou par une solution aqueuse contenant 200 mg/ml de glycine (essai VII).

Les résultats obtenus sont reproduits au tableau V:

TABLEAU V

| Essai | Echantillon | Concentration en pénicilline G (en U.I./ml) | Coloration |
|---|---|---|---|
| | témoin | 0 | |
| I | | | jaune très intense |
| II | | | jaune très intense |
| III | | | jaune très intense |
| IV | | | jaune très intense |
| V | | | jaune très intense |
| VI | | | jaune très intense |
| VII | | | jaune très intense |
| | 1 | 0,003 | |
| I | | | jaune pâle |
| II | | | jaune pâle |
| III | | | jaune pâle |
| IV | | | jaune pâle |
| V | | | jaune pâle |
| VI | | | jaune pâle |
| VII | | | jaune pâle |

TABLEAU V (suite)

| Essai | Echantillon | Concentration en pénicilline G (en U.I./ml) | Coloration |
|---|---|---|---|
| | 2 | 0,006 | |
| I | | | blanche |
| II | | | blanche |
| III | | | blanche |
| IV | | | blanche |
| V | | | blanche |
| VI | | | blanche |
| VII | | | blanche |
| | blanc | 0 | |
| I | | | blanche |
| II | | | blanche |
| III | | | blanche |
| IV | | | blanche |
| V | | | blanche |
| VI | | | blanche |
| VII | | | blanche |

Ce tableau montre que de façon générale les acides aminés de configuration D et la glycine conviennent pour la mise en oeuvre de l'invention.

Exemple 6.

Cet exemple illustre l'application du procédé de l'invention au dosage de la céfapirine, un antibiotique de la famille des céphalosporines, dans le lait.

Le procédé est réalisé de la même manière qu'à l'exemple 3, mais les échantillons de 3 ml de lait renferment des concentrations connues en céfapirine.

Les résultats obtenus sont reproduits au tableau VI:

TABLEAU VI

| Echantillon | Concentration en céfapirine (en µg/ml) | Coloration |
|---|---|---|
| témoin | 0 | jaune très intense |
| 1 | 0,002 | jaune moyen |
| 2 | 0,003 | jaune pâle |
| 3 | 0,004 | blanche |
| 4 | 0,006 | blanche |
| blanc | 0 | blanche |

Ce tableau montre que le procédé de l'invention permet de détecter visuellement la présence de 0,004 µg/ml de céfapirine dans le lait en 15 minutes.

Exemple 7.

Cet exemple illustre l'application du procédé de l'invention au dosage de faibles concentrations de pénicilline G dans du sérum.

On procède comme à l'exemple 3, mais on remplace les échantillons de 3 ml de lait par des échantillons de 3 ml de sérum renfermant des concentrations connues de pénicilline G.

De plus, après la seconde incubation d'une durée de 5 minutes, les échantillons sont centrifugés et les surnageants sont dilués 10 fois avec de l'eau.

On mesure ensuite la densité optique au spectrophotomètre à 410 nm.

Les résultats obtenus sont reproduits au tableau VII.

Les valeurs de la densité optique mentionnées dans le tableau VII sont obtenues en soustrayant la valeur de la densité optique trouvée pour l'échantillon blanc de celles trouvées respectivement pour l'échantillon témoin et pour les autres échantillons.

TABLEAU VII

| Echantillon | Concentration en pénicilline G (en U.I./ml) | Densité optique (à 410 nm) |
|---|---|---|
| témoin | 0 | 435 |
| 1 | 0,002 | 226 |
| 2 | 0,003 | 175 |
| 3 | 0,004 | 49 |
| 4 | 0,006 | 15 |
| blanc | 0 | 0 |

Ce tableau montre qu'il est possible, en utilisant un spectrophotomètre, de déterminer quantitativement de très faibles concentrations en pénicilline G (aussi faibles que 0,002 U.I./ml) dans un échantillon de sérum.

Exemple 8.

Dans cet exemple, le substrat et le réactif permettant la détermination de l'acide 2-mercaptoalcanoïque sont réunis sous la forme d'un comprimé.

Le procédé est réalisé de la même manière qu'à l'exemple 3. Toutefois, après la première incubation d'une durée de 10 minutes, on ajoute un comprimé qui contient 600 µg d'acide [(N-benzoyl-D-alanyl)thio]-acétique et 600 µg d'acide 5,5'-dithiobis(2-nitrobenzoïque).

La composition globale de ce comprimé est la suivante (en % en poids):

| | |
|---|---|
| Substrat + réactif | 4% |
| Polyéthylèneglycol 6000 | 3% |
| Avicel* | 27% |
| Amidon | 10% |
| Lactose | 55% |
| Aerosil** | 0.5% |
| Stéarate de magnésium | 0.5% |

* cellulose microcristalline
** silice colloïdale

Les résultats obtenus sont repris dans le tableau VIII:

TABLEAU VIII

| Echantillon | Concentration en pénicilline G (en U.I./ml) | Coloration |
|---|---|---|
| témoin | 0 | jaune intense |
| 1 | 0,002 | jaune moyen |
| 2 | 0,003 | jaune pâle |
| 3 | 0,004 | blanche |
| 4 | 0,006 | blanche |
| blanc | 0 | blanche |

Ce tableau montre qu'il est possible d'utiliser le procédé de l'invention en associant le substrat et le réactif pour la détermination de l'acide 2-mercaptoalcanoïque sous la forme d'un comprimé, ce qui présente un net avantage tant au niveau pratique qu'au niveau de la stabilité des réactifs.

## Revendications

**1.** Procédé enzymatique pour la détermination d'antibiotiques à noyau $\beta$-lactame dans un liquide biologique comprenant les stades suivants:

(1) l'incubation dudit liquide biologique avec la D-alanyl-D-alanine-carboxypeptidase soluble produite par Actinomadura R39, cette incubation étant menée dans des conditions permettant à l'antibiotique $\beta$-lactame éventuellement présent dans ledit liquide de réagir avec l'enzyme pour former un complexe enzyme-antibiotique équimoléculaire, inactif et sensiblement irréversible;

(2) l'incubation du mélange obtenu à la fin du stade (1) avec une solution de substrat dans des conditions permettant l'hydrolyse dudit substrat par l'enzyme pour former un composé, dont la quantité est proportionnelle à l'activité enzymatique résiduelle;

(3) la détermination de la quantité du composé formée au stade (2);

(4) la comparaison de la valeur déterminée au stade (3) avec un étalon pour obtenir la concentration de l'antibiotique dans le liquide biologique,

caractérisé en ce que l'incubation au stade (2) est effectuée avec un substrat qui est un thioester de formule générale

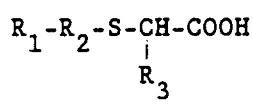

dans laquelle

$R_1$ représente le radical benzoyle, phénylacétyle ou $N^{alpha}$-acétyl-L-lysyle,

$R_2$ représente le radical glycyle ou D-alanyle, et

$R_3$ est un atome d'hydrogène ou un radical méthyle,

de manière à former par hydrolyse un acide 2-mercaptoalcanoïque de formule

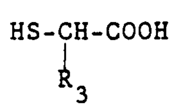

dans laquelle $R_3$ a la signification donnée ci-dessus, et en présence d'un acide aminé qui active l'hydrolyse du substrat par l'enzyme, ledit acide aminé étant choisi parmi un acide aminé de configuration D et la glycine.

**2.** Procédé selon la revendication 1, caractérisé en ce que le substrat est l'acide [(N-benzoyl-D-alanyl)-thio]-acétique.

3. Procédé selon l'une quelconque des revendications 1 et 2, caractérisé en ce que l'acide aminé est choisi dans le groupe consistant en la D-alanine, la D-méthionine, la D-arginine, la D-phénylalanine, la D-sérine, la D-histidine, la D-valine, le D-tryptophane et l'acide D-2-aminobutyrique.

4. Procédé selon la revendication 3, caractérisé en ce que l'acide aminé est la D-alanine.

5. Procédé selon l'une quelconque des revendications 1 à 4, caractérisé en ce que les stades (2) et (3) sont exécutés simultanément.

6. Procédé selon l'une quelconque des revendications 1 à 5, caractérisé en ce que le liquide biologique est choisi parmi le lait, le sérum, l'urine, la salive, les extraits de viande, les liquides de fermentation et les solutions aqueuses tamponnées.

7. Procédé selon l'une quelconque des revendications 1 à 6, caractérisé en ce que l'antibiotique à déterminer est choisi parmi la benzylpénicilline, l'ampicilline, la phénoxyméthylpénicilline, la carbénicilline, la méthicilline, l'oxacilline, la cloxacilline, la céphalosporine C, la céfaloglycine, la céfalothine, la céfalexine et la céfapirine.

8. Procédé selon l'une quelconque des revendications 1 à 7, caractérisé en ce que la quantité d'acide 2-mercaptoalcanoïque de formule

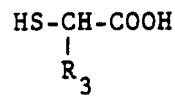

est déterminée par incubation du mélange obtenu au stade (2) avec l'acide 5,5'-dithiobis(2-nitrobenzoïque) de manière à produire une coloration dont l'intensité est fonction de la quantité d'acide 2-mercaptoalcanoïque.

9. Trousse d'essai pour la détermination d'antibiotiques à noyau $\beta$-lactame dans un liquide biologique comprenant:

(1) une quantité déterminée de D-alanyl-D-alanine-carboxypeptidase soluble produite par Actinomadura R39;

(2) une quantité déterminée d'un substrat qui est un thioester de formule générale

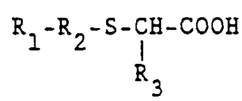

dans laquelle

- $R_1$ représente le radical benzoyle, phénylacétyle ou $N^{alpha}$-acétyl-L-lysyle,
- $R_2$ représente le radical glycyle ou D-alanyle, et
- $R_3$ est un atome d'hydrogène ou un radical méthyle,

(3) une quantité déterminée d'un acide aminé de configuration D ou de glycine,

(4) un réactif permettant la détermination d'un acide 2-mercaptoalcanoïque de formule

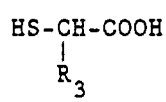

dans laquelle $R_3$ à la signification donnée ci-dessus; et

(5) éventuellement un étalon, par rapport auquel les résultats d'essais réalisés avec les réactifs (1), (2), (3) et (4) peuvent être comparés.

**10.** Trousse d'essai selon la revendication 9, caractérisée en ce que le substrat est l'acide [(N-benzoyl-D-alanyl)thio]-acétique.

**11.** Trousse d'essai selon l'une quelconque des revendications 9 et 10, caractérisée en ce que l'acide aminé de configuration D est choisi dans le groupe consistant en la D-alanine, la D-méthionine, la D-arginine, la D-phénylalanine, la D-sérine, la D-histidine, la D-valine, le D-tryptophane et l'acide D-2-aminobutyrique.

**12.** Trousse d'essai selon la revendication 11, caractérisée en ce que l'acide aminé de configuration D est la D-alanine.

**13.** Trousse d'essai selon l'une quelconque des revendications 9 à 12, caractérisée en ce que le réactif (4) est l'acide 5,5'-dithiobis(2-nitrobenzoïque).

**14.** Trousse d'essai selon l'une quelconque des revendications 9 à 13, caractérisée en ce que le substrat et le réactif (4) sont réunis sous la forme d'un comprimé.

**Claims**

**1.** Enzymatic process for the determination of $\beta$-lactam antibiotics in a biological liquid comprising the following steps:

(1) incubation of the said biological liquid with the soluble D-alanyl-D-alanine-carboxypeptidase produced by Actinomadura R39, this incubation being conducted under conditions allowing the $\beta$-lactam antibiotic, which may be present in the said liquid, to react with the enzyme to form an inactive and substantially irreversible equimolecular enzyme-antibiotic complex;

(2) incubation of the mixture obtained at the end of step (1) with a substrate solution under conditions allowing the substrate to be hydrolysed by the enzyme to form a compound in an amount proportional to the residual enzymatic activity;

(3) determination of the amount of the compound formed in step (2);

(4) comparison of the value determined in step (3) with a standard to obtain the concentration of the antibiotic in the biological liquid,

characterised in that the incubation in step (2) is carried out with a substrate which is a thioester of the general formula

$$R_1-R_2-S-\underset{\displaystyle R_3}{\underset{|}{CH}}-COOH$$

wherein

$R_1$ represents a benzoyl, phenylacetyl or $N^{alpha}$-acetyl-L-lysyl radical

$R_2$ represents a glycyl or D-alanyl radical, and

$R_3$ is a hydrogen atom or a methyl radical,

so as to form, by hydrolysis, a 2-mercaptoalkanoic acid of the formula

$$HS-\underset{\displaystyle R_3}{\underset{|}{CH}}-COOH$$

wherein $R_3$ has the meaning given above, and in the presence of an amino acid which activates the hydrolysis of the substrate by the enzyme, the said amino acid being selected from a D-amino acid and glycine.

**2.** Process according to claim 1, characterised in that the substrate is [(N-benzoyl-D-alanyl)thio]-acetic acid.

**3.** Process according to any of claims 1 and 2, characterised in that the amino acid is selected from the group consisting of D-alanine, D-methionine, D-arginine, D-phenylalanine, D-serine, D-histidine, D-valine, D-tryptophan and D-2-aminobutyric acid.

**4.** Process according to claim 3, characterised in that the amino acid is D-alanine.

**5.** Process according to any of claims 1 to 4, characterised in that steps (2) and (3) are carried out simultaneously.

**6.** Process according to any of claims 1 to 5, characterised in that the biological liquid is selected from milk, serum, urine, saliva, meat extracts, fermentation liquids and buffered aqueous solutions.

**7.** Process according to any of claims 1 to 6, characterised in that the antibiotic to be determined is selected from benzylpenicillin, ampicillin, phenoxymethylpenicillin, carbenicillin, methicillin, oxacillin, cloxacillin, cephalosporin c, cephaloglycin, cephalothin, cephalexin and cephapirin.

**8.** Process according to any of claims 1 to 7, characterised in that the amount of 2-mercaptoalkanoic acid of the formula

```
HS-CH-COOH
   |
   R
    3
```

is determined by incubation of the mixture obtained in step (2) with 5,5'-dithiobis(2-nitrobenzoic) acid so as to produce a coloration, the intensity of which is a function of the amount of 2-mercaptoalkanoic acid.

**9.** Test set for the determination of $\beta$-lactam antibiotics in a biological liquid, comprising:

(1) a definite amount of soluble D-alanyl-D-alanine-carboxypeptidase produced by <u>Actinomadura R39</u>;

(2) a definite amount of a substrate which is a thioester of the general formula

```
R -R -S-CH-COOH
 1  2    |
         R
          3
```

wherein

$R_1$ represents a benzoyl, phenylacetyl or $N^{alpha}$-acetyl-L-lysyl radical,

$R_2$ represents a glycyl or D-alanyl radical, and

$R_3$ is a hydrogen atom or a methyl radical,

(3) a definite amount of a D-amino acid or of glycine,

(4) a reagent allowing the determination of a 2-mercaptoalkanoic acid of the formula

```
HS-CH-COOH
   |
   R
    3
```

wherein $R_3$ has the meaning given above; and

(5) if appropriate, a standard with which the results of tests carried out with reagents (1), (2), (3) and (4) may be compared.

**10.** Test set according to claim 9, characterised in that the substrate is [(N-benzoyl-D-alanyl)thio]-acetic acid.

**11.** Test set according to any of claims 9 and 10, characterised in that the D-amino acid is selected from the group consisting of D-alanine, D-methionine, D-arginine, D-phenylalanine, D-serine, D-histidine, D-valine, D-tryptophan and D-2-aminobutyric acid.

**12.** Test set according to claim 11, characterised in that the D-amino acid is D-alanine.

**13.** Test set according to any of claims 9 to 12, characterised in that the reagent (4) is 5,5'-dithiobis(2-nitrobenzoic) acid.

**14.** Test set according to any of claims 9 to 13, characterised in that the substrate and the reagent (4) are combined in the form of a tablet.

**Patentansprüche**

**1.** Enzymatisches Verfahren zur Bestimmung von einen $\beta$-Lactam-Kern enthaltende Antibiotika in einer biologischen Flüssigkeit, das die folgenden Stufen umfasst:

(1) Inkubation der genannten biologischen Flüssigkeit mit der löslichen D-Alanyl-D-alanin-carboxypeptidase hergestellt von Actinomadura R39, wobei diese Inkubation unter Bedingungen durchgeführt wird, die eine Reaktion des gegebenenfalls in der Flüssigkeit vorhandenen $\beta$-Lactam-Antibiotikums mit dem Enzym zur Bildung eines inaktiven und merklich irreversibelen äquimolaren Enzym-Antibiotikum-Komplexes erlauben;

(2) Inkubation des am Ende der Stufe (1) erhaltenen Gemisches mit einer Substratlösung unter Bedingungen, die die Hydrolyse des Substrats durch das Enzym ermöglichen zur Bildung einer Verbindung, deren Menge der restlichen enzymatischen Aktivität entspricht;

(3) Bestimmung der Menge der in Stufe (2) gebildeten Verbindung;

(4) Vergleich des in Stufe (3) bestimmten Wertes mit einem Standard, um die Konzentration des Antibiotikums in der biologischen Flüssigkeit zu erhalten,

dadurch gekennzeichnet, daß die Inkubation in Stufe (2) mit einem Substrat ausgeführt wird, das ein Thioester der allgemeinen Formel

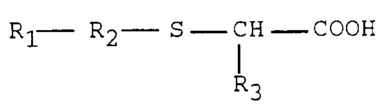

ist, in welcher

$R_1$ den Benzoyl-, Phenylacetyl- oder $N^{\text{alpha}}$-Acetyl-L-lysylrest darstellt,

$R_2$ den Glycyl- oder D-Alanylrest darstellt, und

$R_3$ ein Wasserstoffatom oder ein Methylrest ist,

wobei durch Hydrolyse eine 2-Mercaptoalkansäure der Formel

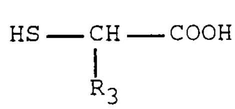

gebildet wird, in welcher $R_3$ die oben angegebene Bedeutung besitzt, und in Gegenwart einer Aminosäure, die die Hydrolyse des Substrats durch das Enzym aktiviert, wobei die genannte Aminosäure ausgewählt wird aus einer Aminosäure mit D-Konfiguration und Glycin.

**2.** Verfahren nach Anspruch 1, dadurch gekennzeichnet, daß das Substrat [(N-Benzoyl-D-alanyl)thio]-essigsäure ist.

**3.** Verfahren nach irgendeinem der Ansprüche 1 und 2, dadurch gekennzeichnet, daß die Aminosäure ausgewählt wird aus der Gruppe aus D-Alanin, D-Methionin, D-Arginin, D-Phenylalanin, D-Serin, D-Histidin, D-Valin, D-Tryptophan und D-2-Aminobuttersäure.

**4.** Verfahren nach Anspruch 3, dadurch gekennzeichnet, daß die Aminosäure D-Alanin ist.

**5.** Verfahren nach irgendeinem der Ansprüche 1 bis 4, dadurch gekennzeichnet, daß die Stufen (2) und (3) gleichzeitig durchgeführt werden.

**6.** Verfahren nach irgendeinem der Ansprüche 1 bis 5, dadurch gekennzeichnet, daß die biologische Flüssigkeit ausgewählt wird aus Milch, Serum, Urin, Speichel, Fleischextrakten, Fermentationsflüssigkeiten und wäßrigen Pufferlösungen.

**7.** Verfahren nach irgendeinem der Ansprüche 1 bis 6, dadurch gekennzeichnet, daß das zu bestimmende Antibiotikum ausgewählt wird aus Benzylpenicillin, Ampicillin, Phenoxymethylpenicillin, Carbenicillin, Methicillin, Oxacillin, Cloxacillin, Cephalosporin C, Cefaloglycin, Cefalothin, Cefalexin und Cefapirin.

**8.** Verfahren nach irgendeinem der Ansprüche 1 bis 7, dadurch gekennzeichnet, daß die Menge der 2-Mercaptoalkansäure der Formel

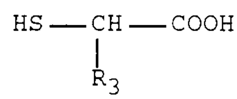

durch Inkubation des von Stufe (2) erhaltenen Gemisches mit 5,5'-Dithiobis(2-nitrobenzoesäure) bestimmt wird, wobei eine Färbung erzeugt wird, deren Intensität eine Funktion der Menge an 2-Mercaptoalkansäure ist.

**9.** Testbesteck zur Bestimmung von einen β-Lactam-Kern enthaltende Antibiotika in einer biologischen Flüssigkeit, das umfaßt:

(1) eine bestimmte Menge der löslichen D-Alanyl-D-alanin-carboxypeptidase, hergestellt von Actinomadura R39;

(2) eine bestimmte Menge eines Substrats, das ein Thioester der allgemeinen Formel

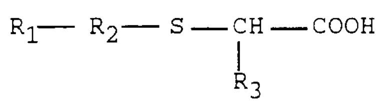

ist, in welcher

$R_1$ den Benzoyl-, Phenylacetyl- oder $N^{alpha}$-Acetyl-L-lysylrest darstellt,

$R_2$ den Glycyl- oder D-Alanylrest darstellt und

$R_3$ ein Wasserstoffatom oder ein Methylrest ist,

(3) eine bestimmte Menge einer Aminosäure mit D-Konfiguration oder Glycin,

(4) ein Reagenz, das die Bestimmung einer 2-Mercaptoalkansäure der Formel

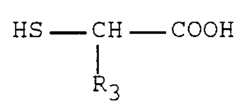

erlaubt, in welcher $R_3$ die oben angegebene Bedeutung besitzt; und

(5) gegebenenfalls einen Standard, mit dem die mit den Reagenzien (1), (2), (3) und (4) durchgeführten Versuchsergebnisse verglichen werden können.

**10.** Testbesteck nach Anspruch 9, dadurch gekennzeichnet, daß das Substrat [(N-Benzoyl-D-alanyl)thio]-essigsäure ist.

**11.** Testbesteck nach irgendeinem der Ansprüche 9 und 10, dadurch gekennzeichnet, daß die Aminosäure mit D-Konfiguration ausgewählt wird aus der Gruppe aus D-Alanin, D-Methionin, D-Arginin, D-Phenylalanin, D-Serin, D-Histidin, D-Valin, D-Tryptophan und D-2-Aminobuttersäure.

**12.** Testbesteck nach Anspruch 11, dadurch gekennzeichnet, daß die Aminosäure mit D-Konfiguration D-Alanin ist.

**13.** Testbesteck nach irgendeinem der Ansprüche 9 bis 12, dadurch gekennzeichnet, daß das Reagenz (4) 5,5'-Dithiobis(2-nitrobenzoesäure) ist.

**14.** Testbesteck nach irgendeinem der Ansprüche 9 bis 13, dadurch gekennzeichnet, daß das Substrat und das Reagenz (4) in Form einer Tablette vereinigt sind.